# FASCICULE DE BREVET EUROPEEN

(11) **EP 1 233 710 B1**
(45) Date de publication et mention de la délivrance du brevet: **02.03.2005**
(21) Numéro de dépôt: 00985387.0
(22) Date de dépôt: 01.12.2000
(51) Int. Cl.: A61B 17/70

(54) **ENSEMBLE DE CONNEXION POUR LE DOMAINE DE L'OSTEOSYNTHESE RACHIDIENNE**
FIXIERANORDNUNGE FÜR WIRBELSÄULENOSTEOSYTHESE
CONNECTING ASSEMBLY FOR SPINAL OSTEOSYNTHESIS

(30) Priorité: 03.12.1999 FR 9915295
(43) Date de publication de la demande: 28.08.2002
(73) Titulaire: Spinevision S.A., 75012 Paris (FR)
(72) Inventeur: PETIT, Dominique, F-62180 Verton (FR); BETTE, Stéphane, F-75011 Paris (FR); CAZIN, Muriel, F-77600 Bussy-Saint-Georges (FR)
(74) Mandataire: Breese, Pierre
(86) Numéro de dépôt international: PCT/FR2000/003365
(87) Numéro de publication internationale: WO 2001/039677

(56) Documents cités:
- DE-U- 9 215 561
- FR-A- 2 776 500
- US-A- 5 254 118
- US-A- 5 562 661

## Description

La présente invention a pour objectif une application dans le domaine de l'ostéosynthèse rachidienne.

La présente invention se rapporte plus particulièrement à un ensemble de connexion pour l'ostéosynthèse rachidienne comportant un moyen d'ancrage osseux comprenant et une zone de connexion destinée à coopérer avec un moyen de connexion.

L'art antérieur connaît des ensembles de connexion et notamment le modèle d'utilité allemand numéro 92 15 561, montrant les caractéristiques du préambule de la revendication 1.

L'inconvénient majeur des ensembles de connexion de l'art antérieur réside dans le fait qu'ils n'offrent que peu de possibilité de mouvement des différents éléments les uns par rapport aux autres.

La présente invention concerne dans son acception la plus générale un ensemble de connexion selon la revendication 1.

Avantageusement, le moyen de connexion est serti dans le connecteur de façon à solidariser les deux pièces tout en laissant une rotation du moyen de connexion dans le connecteur.

Selon une variante, le connecteur présente un cône d'entrée dans le connecteur pour le passage de l'ancillaire pour l'entraînement en rotation du moyen de connexion.

De préférence, le débattement angulaire du connecteur sur le moyen de connexion est de 30 degrés environ quand l'ancillaire d'entraînement en rotation du moyen de connexion est en place.

Selon un mode de réalisation préféré, le moyen de connexion présente des fentes usinées dans la partie sphérique du moyen de connexion de façon à créer une déformation lors du serrage définitif du système. Lesdites fentes sont de préférence longitudinales.

Ledit moyen de connexion comporte de préférence également dans sa partie inférieure une jupe. Cette jupe est filetée sur son extrémité inférieure pour faciliter sa pénétration dans l'os.

Ledit un moyen d'ancrage osseux comporte de préférence dans sa partie supérieure une partie de pré-guidage dudit moyen de connexion afin de permettre de réaliser un bon alignement du moyen de connexion avec la vis.

Le moyen de connexion peut être constitué par un écrou.

Le connecteur est pourvu d'au moins un emplacement pour recevoir un élément de liaison.

Le connecteur peut comporter un emplacement de blocage débouchant dans la cavité formant le logement de la forme sphérique et dans l'emplacement recevant l'élément de liaison.

L'ensemble de connexion selon l'invention peut comporter un cylindre de blocage qui peut être introduit dans ledit emplacement de blocage. Ce cylindre de blocage peut être remplacé par un élément de liaison.

L'emplacement de blocage peut en outre déboucher également dans une direction sensiblement perpendiculaire pour l'introduction d'un bouchon de blocage. Le bouchon de blocage peut comporter un téton destiné à coopérer avec un emplacement ménagé dans ledit cylindre de réglage.

L'emplacement pour recevoir un élément de liaison peut être en forme de oblong dans le cas d'un connecteur de type fermé, ou en forme de « U » débouchante sur une des faces dans le cas d'un connecteur de type ouvert.

Le connecteur peut être pourvu de deux emplacements pour recevoir un élément de liaison formé par une tige coudée formant un U fermé à ses extrémités et en ce que l'élément de liaison présente une cavité de forme complémentaire à la forme sphérique.

Une partie filetée, en forme d'hexagone, est de préférence aménagée à l'extrémité supérieure du moyen de connexion, afin de permettre de visser un écrou secondaire.

La présente invention sera mieux comprise à la lecture de la description d'un exemple non limitatif de réalisation qui suit, se référant aux dessins annexés où :
- la figure 1 représente une vue en perspective d'un ensemble de connexion dans sa version de base avec un moyen d'ancrage osseux vis ;
- la figure 2 représente une vue éclatée de l'ensemble de connexion de la figure 1 ;
- la figure 3 représente une vue en coupe partielle de l'ensemble de connexion de la figure 1 ;
- la figure 4 représente une vue en perspective d'un écrou selon l'invention avec jupe et fentes longitudinales ;
- la figure 5 représente une vue partielle en perspective d'un écrou selon la figure 4 monté sur un connecteur ;
- la figure 6 représente une vue en perspective d'une vis comportant une partie de pré-guidage et/ou une partie de rappel lisse, ainsi qu'une partie de connexion lisse ;
- la figure 7 représente une vue en perspective d'une vis comportant une partie de pré-guidage lisse, une partie de rappel filetée et une partie de connexion filetée confondue avec le filetage osseux ;
- la figure 8 représente une vue en perspective d'une vis comportant une partie de pré-guidage lisse, une partie de rappel filetée et une partie de connexion filetée non confondue avec le filetage osseux ;
- la figure 9 représente une vue en perspective d'une vis comportant une partie de pré-guidage lisse et une partie de connexion filetée non confondue avec le filetage osseux ;
- la figure 10 représente une vue en perspective d'un ensemble de connexion avec un système de blocage entre le moyen de connexion et la tige de connexion ;
- la figure 11 représente une vue en coupe de l'ensemble de connexion de la figure 10 avec un cylindre de blocage ;
- la figure 12 représente une vue en perspective de l'ensemble de connexion de la figure 10 avec un deuxième élément de connexion ;
- la figure 13 représente une vue en perspective d'un ensemble de connexion dans une variante avec une tige de liaison coudée formant un U fermé à ses extrémités ;
- la figure 14 représente une vue éclatée de l'ensemble de connexion de la figure 13 ;
- la figure 15 représente une vue partielle en coupe de l'ensemble de connexion de la figure 13 ;
- la figure 16 représente une vue en perspective d'un moyen d'ancrage osseux crochet ; et
- la figure 17 représente une vue en perspective d'un ensemble de connexion avec un moyen d'ancrage osseux crochet.

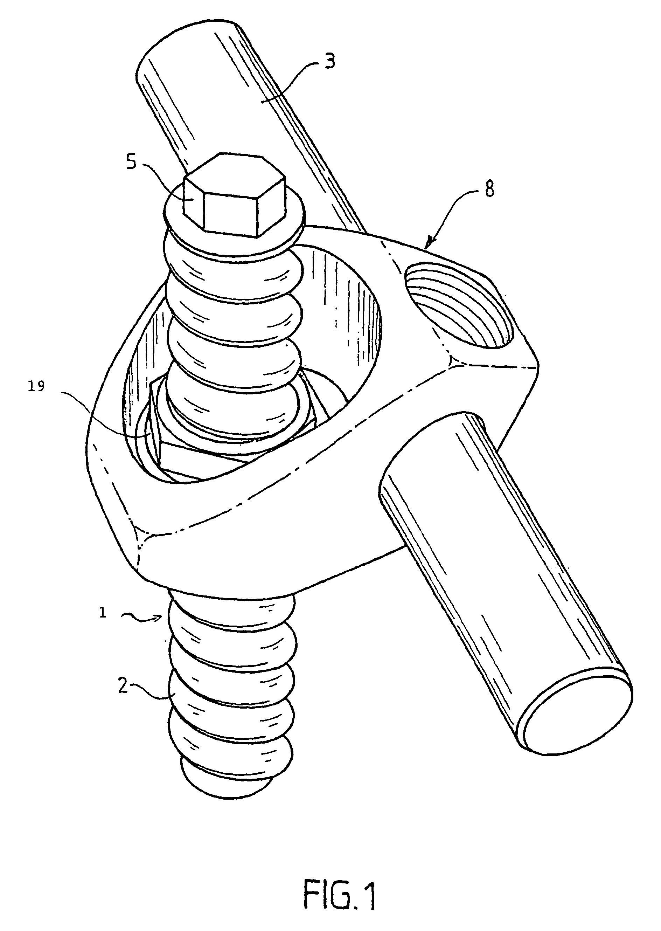

La présente invention se rapporte à un ensemble de connexion, illustré dans sa version de base figure 1, pour l'ostéosynthèse rachidienne comportant un moyen d'ancrage osseux comprenant une zone de connexion (29) destinée à coopérer avec un moyen de connexion.

Le moyen d'ancrage osseux peut être constitué par une vis (1) comportant au moins un filetage osseux (2).

La vis (1) comporte en outre une zone de connexion (29) destinée à coopérer avec ledit moyen de connexion.

Le filetage de type osseux (2) présente une fonction principale : celle d'assurer l'encrage dans l'os. Il peut éventuellement présenter une fonction secondaire : celle de recevoir le système de connexion pour un élément de liaison lorsque la zone de connexion (29) est confondue avec la partie supérieure du filetage osseux (2).

Sur l'extrémité supérieure de la vis (1), un système d'entraînement (5) en rotation est prévu. Ce système par exemple un hexagone, a deux fonctions : Celle de permettre l'entraînement en rotation de la vis (1) lors de la pénétration dans l'os de celle-ci, mais également un rôle de blocage en rotation lors du serrage final du mécanisme pour éviter une pénétration plus importante de la vis (1) dans l'os.

Le moyen de connexion permet de réaliser une butée longitudinale le long de la vis (1) soit directement pour l'élément de liaison (3') lorsque, par exemple, celui-ci est une tige coudée formant un U fermé à ses extrémités, soit par le biais d'un connecteur (8).

Le moyen de connexion peut être constitué par un écrou (19). Dans ce cas, dont le taraudage (7) de l'écrou (19) correspond au filetage de la zone de connexion (29) ; c'est à dire, dans la version illustrée figures 1, 2 et 3, au filetage osseux (2) de la vis (1). L'écrou (19) est vissé sur la partie non enfouie de l'os de la vis.

L'écrou (19) est dans sa partie inférieure de forme sphérique (20). Cette forme sphérique est destinée à laisser un positionnement libre de l'écrou (19) sur le connecteur (8) où est prévu un logement du même type ou sur l'élément de liaison (3). Cette forme sphérique (20) sert également de butée de positionnement longitudinal avec le connecteur (8) ou avec l'élément de liaison (3). Dans l'application préférentielle, le moyen de connexion est serti dans le connecteur (8) ou dans l'élément de liaison (3) de façon à solidariser les deux pièces tout en laissant une rotation du moyen de connexion sur le connecteur (8) ou sur l'élément de liaison (3).

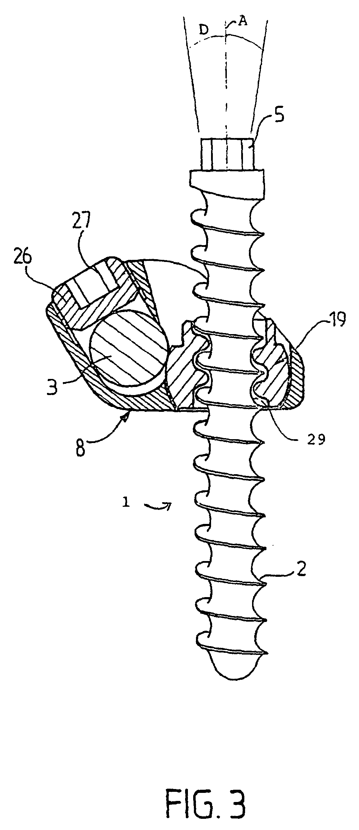

Un système d'entraînement (21) par exemple un hexagone extérieur est également prévu sur le moyen de connexion au-dessus de sa partie sphérique (20) de façon à permettre son réglage en hauteur ainsi que celui du connecteur (8) ou de l'élément de liaison (3') le long de la vis (1). En conséquence de la possibilité de rotation du connecteur sur le moyen de connexion, un cône (22) d'entrée dans le connecteur (8) est prévu pour le passage de l'ancillaire pour l'entraînement en rotation du moyen de connexion. A titre d'exemple, le débattement angulaire D de l'axe A du connecteur sur le moyen de connexion est dans l'application préférentielle de 30 degrés quand l'ancillaire d'entraînement en rotation du moyen de connexion est en place, comme illustré figure 3.

Des fentes (23) sont usinées dans la partie sphérique du moyen de connexion de façon à créer une déformation lors du serrage définitif du système. Cette déformation a pour but de bloquer en rotation la vis (1).

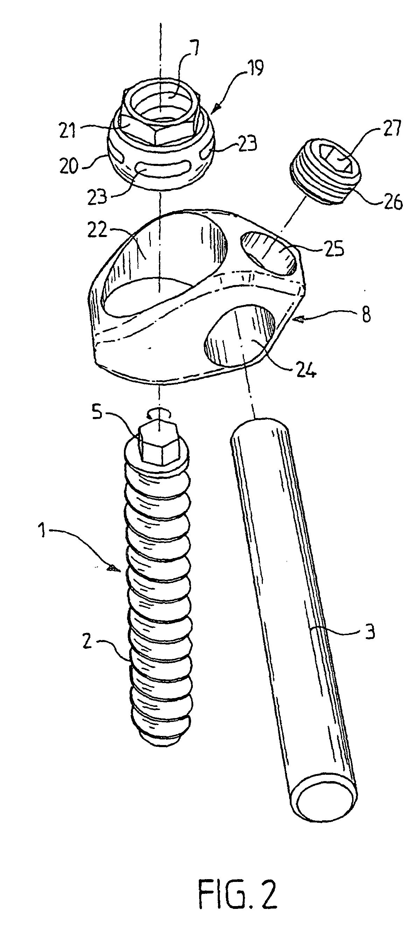

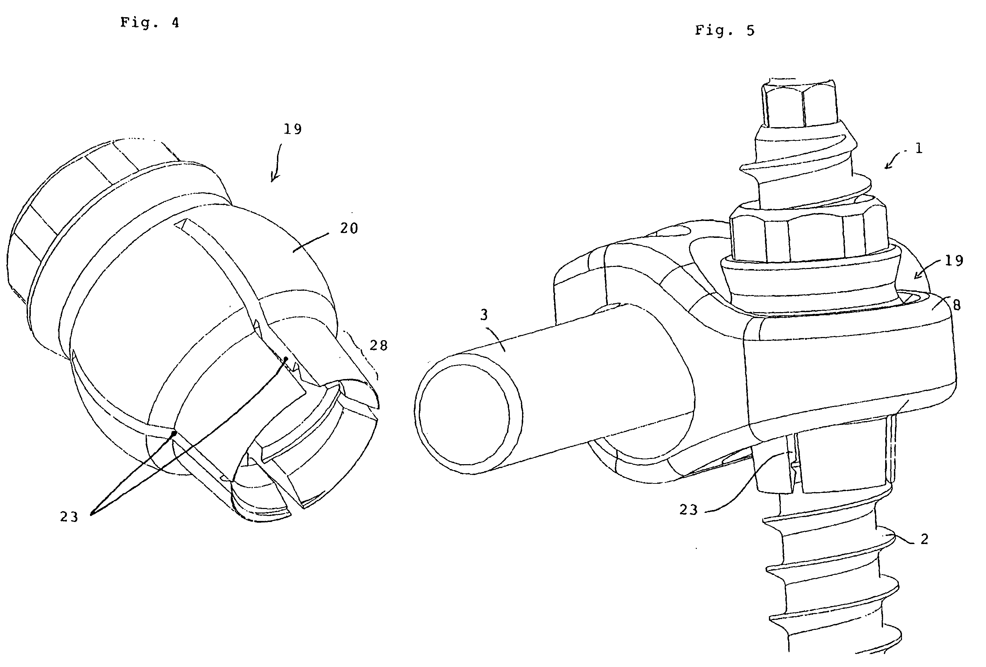

Les fentes (23) peuvent être positionnées transversalement, comme illustré figure 2 ou longitudinalement, comme illustré figure 4.

Les fentes longitudinales sont de préférence débouchantes dans la partie inférieure de la forme sphérique (20). Elles peuvent être au nombre de une, deux, trois, quatre, cinq, ou plus.

Le moyen de connexion comporte dans sa partie inférieure une jupe (28) également visible figure 4, ainsi que figure 5. Cette jupe (28) permet d'avoir une transition mécanique entre la vis (1) et la forme sphérique (20). En effet, une transition trop franche favoriserait une rupture de la vis à la base de la forme sphérique (20) lors d'efforts dynamiques. La jupe (28) permet ainsi de mieux répartir la contrainte d'encastrement de la vis dans l'écrou.

Cette jupe (28) peut être filetée sur son extrémité pour faciliter sa pénétration dans l'os.

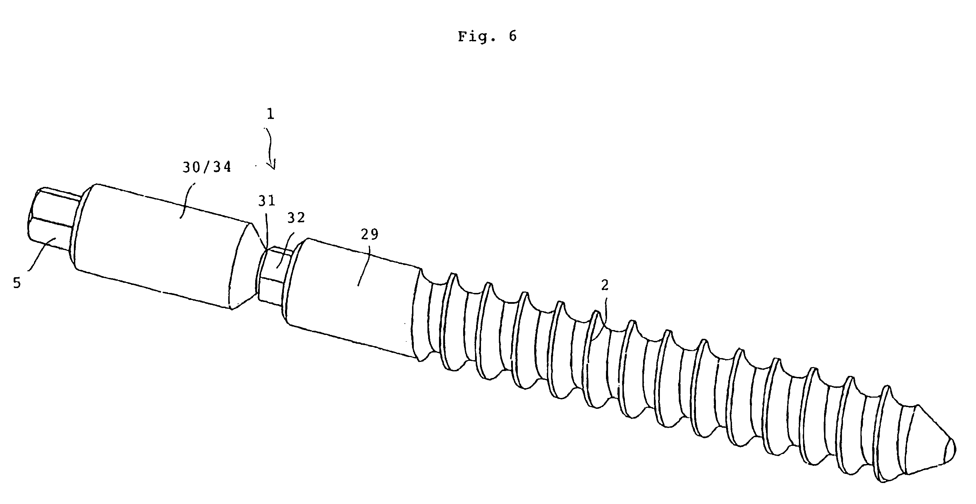

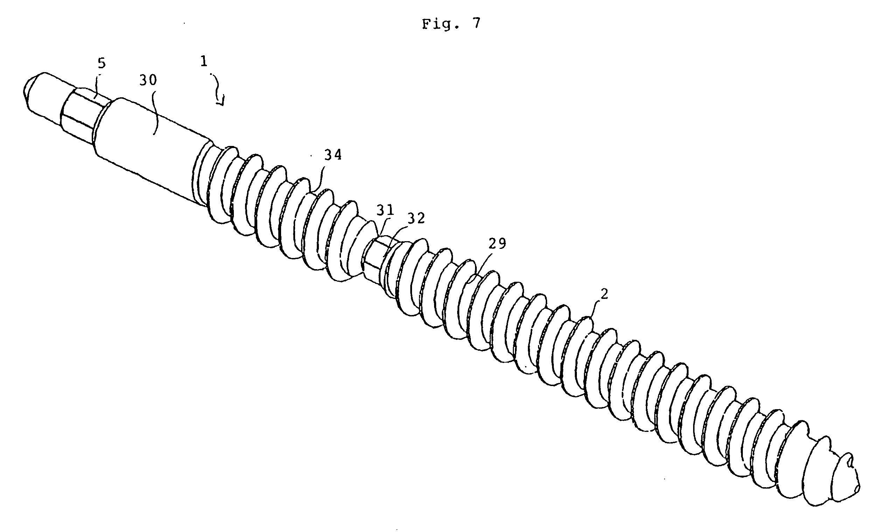

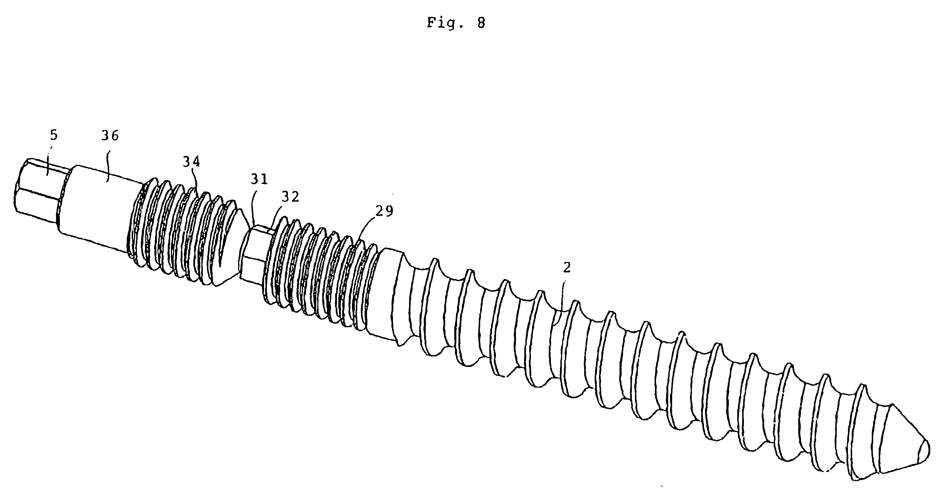

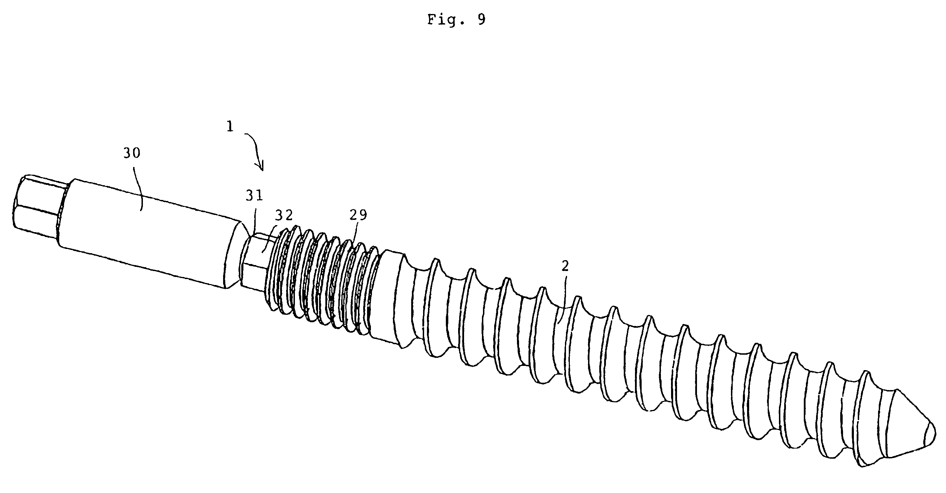

La zone de connexion (29) de la vis (1) peut être ménagée dans la parie supérieure du filetage osseux et présenter un filetage identique au filetage osseux, comme illustré figure 7, mais elle peut également présenter un filetage différent, comme illustré figures 8 et 9, ou encore ne pas être filetée, comme illustré figure 6.

Dans ce dernier cas, le moyen de connexion présente par conséquent une paroi intérieure lisse et ne constitue pas un écrou.

Dans une variante, la vis (1) comporte dans sa partie supérieure une partie lisse (30) de pré-guidage, afin d'assurer le bon alignement du moyen de connexion lors de la mise en place du moyen de connexion, a fortiori lorsqu'il s'agit d'un écrou (19).

Cette partie de pré-guidage (30) est à paroi lisse, comme illustré aux figures 6 à 9. Sin diamètre extérieur est sensiblement équivalent à celui du diamètre intérieur du moyen de connexion pour assurer un guidage longitudinal le plus linéaire possible (sans pente angulaire).

La partie lisse (30) de pré-guidage peut également servir de zone de rappel (34) dans le cas de traitement de spondylolisthésis.

Cette zone de rappel (34) peut également être filetée avec un filetage identique au filetage osseux (2), comme illustré figure 7, ou avec un filetage différent, comme illustré figure 8.

La vis (1) peut également être munie d'une fente (31) facilitant la désolidarisation du haut de la vis (1) comportant la partie lisse (30) de pré-guidage et/ou la zone de rappel (34). Sous cette fente (31) circulaire, un système d'entraînement (32) est usiné qui après la rupture devient le système d'entraînement de la vis (1), particulièrement pour l'ablation de l'ensemble de connexion, comme illustré aux figures 7 à 9.

Dans la version de base, le connecteur (8) est pourvu d'un seul emplacement pour recevoir un élément de liaison (3) formé par une tige. Cet emplacement peut être en forme de oblong (24) dans le cas d'un connecteur (8) de type fermé, soit en forme de « U » débouchante sur une des faces dans le cas d'un connecteur de type ouvert. Dans le cas d'un connecteur (8) de type fermé, la tige doit être enfilée dans celui-ci, tandis que dans le cas d'un connecteur ouvert, la tige (3) peut être introduite sur le connecteur postérieurement ou latéralement.

L'emplacement (24) de réception de la tige (3) est prévu de manière à ce que la tige (3) puisse venir en appui sur la forme sphérique (20) de l'écrou (19), comme illustré figure 3.

Il est donc entendu que la tige (3) par rapport à la vis (1) est libre dans trois axes de rotations et dans deux directions de translations :
- Rotation du connecteur (8) et donc de la tige (3) autour du moyen de fixation dans deux axes perpendiculaires à la vis (1) ;
- Rotation du connecteur (8) et donc de la tige (3) autour du moyen de fixation dans un axe identique à celui de la vis (1) ;
- Translation de la tige (3) dans le connecteur (8) suivant l'axe de celle-ci ;
- Translation du connecteur (8) et donc de la tige (3) le long de la vis (1) grâce à la possibilité de réglage du moyen de fixation.

En outre, la rotation de la tige (3) sur elle-même peut offrir un degré de liberté supplémentaire à l'ensemble de connexion.

Le connecteur (8) possède également un emplacement de blocage (25) pour recevoir un système de blocage. Dans l'application de base, ce système de blocage est un écrou (26) pourvu d'un système d'entraînement (27) pour appliquer un couple suffisant à la bonne tenue mécanique de l'ensemble, comme illustré figure 3.

Le blocage d'une telle connexion est assurée par la pression de l'élément de liaison (3) sur la forme sphérique (20). L'élément de liaison (3) étant solidaire du connecteur (8) par la pression opérée par l'écrou (26), les degrés de liberté sont alors tous figés.

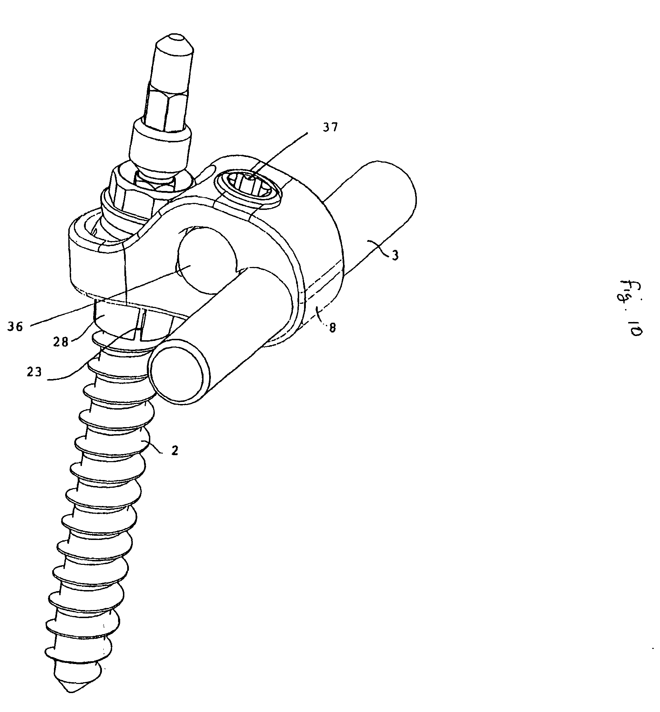

Dans une variante, ledit connecteur (8) comporte un emplacement de blocage (25) évolué, dans lequel peut être introduit un cylindre de blocage (36), ledit emplacement de blocage (25) débouchant également dans une direction sensiblement perpendiculaire pour l'introduction d'un bouchon de blocage (37), comme illustré figure 10.

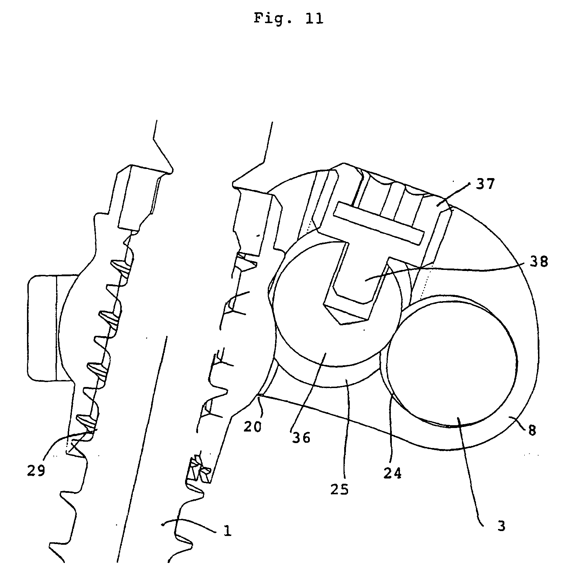

En outre, ledit bouchon de blocage (37) comporte un téton (38) destiné à coopérer avec un emplacement ménagé dans ledit cylindre de blocage (36), afin d'empêcher que le cylindre de blocage (36) ne se désolidarise du connecteur lorsque l'ensemble n'est pas encore serré, comme illustré figure 11.

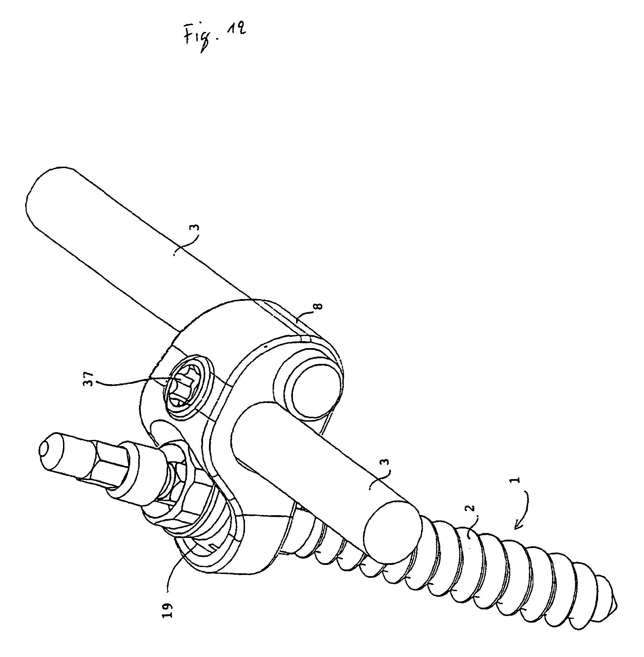

Le cylindre de blocage (36) peut être remplacé par un élément de liaison (3), afin d'offrir une possibilité supplémentaire de connexion, comme illustré figure 12.

Dans cette version, le serrage de l'ensemble est assuré par la force qui est opérée à la fois sur l'élément de liaison (3) et sur la partie sphérique (20) par le cylindre de connexion (36) ou par le second élément de liaison (3).

Il est nécessaire de prévoir une pluralité de connecteurs, afin de pouvoir choisir celui dont la distance entre la vis et l'élément de liaison ou les éléments de liaison est adéquat.

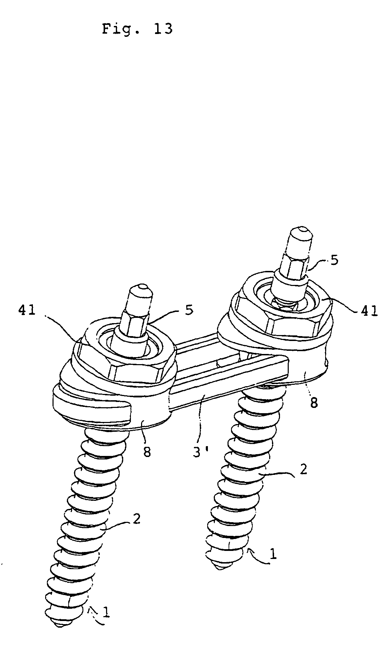

Dans une autre version, le connecteur (8) est pourvu de deux emplacements pour recevoir un élément de liaison (3') formé par une tige coudée formant un U fermé à ses extrémités, comme illustré figure 13 et l'élément de liaison (3') présente une cavité de forme complémentaire à la forme sphérique (20).

La tige est courbée sur elle-même, de façon à former un anneau de section transversale oblongue assimilable à une plaque dans le domaine de l'ostéosynthèse.

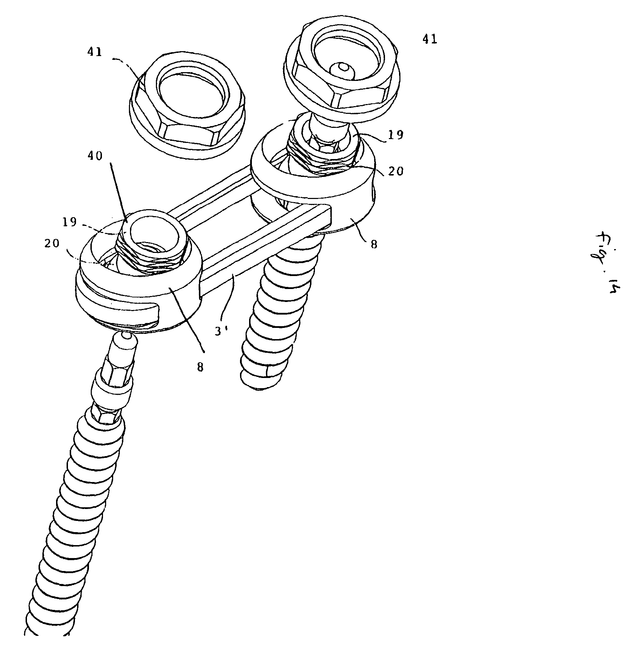

Une partie filetée (40), en forme d'hexagone, est aménagée à l'extrémité supérieure de l'écrou (19), comme illustré figure 14, afin de permettre de visser un écrou secondaire (41) et serrer l'ensemble.

Le connecteur (8) est assimilable à une rondelle présentant une cavité pour le passage de l'élément de liaison.

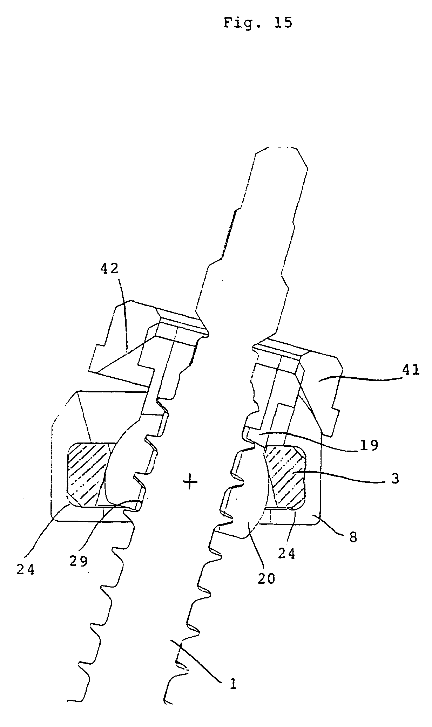

Un chanfrein (42), illustré figure 15 est aménagé sur l'écrou secondaire (41) ; Ce chanfrein est destiné à coopérer avec la partie supérieure extérieure du connecteur (8).

Dans cette version, le moyen de fixation est serti sur le connecteur (8), emprisonnant l'élément de liaison (3'), de façon à solidariser les trois pièces tout en autorisant une rotation du moyen de fixation dans le connecteur (8) et une translation de l'élément de liaison (3') dans ce connecteur (8).

Le serrage final est réalisé en vissant l'écrou secondaire (41) sur le moyen de fixation de manière à figer l'ensemble des degrés de liberté.

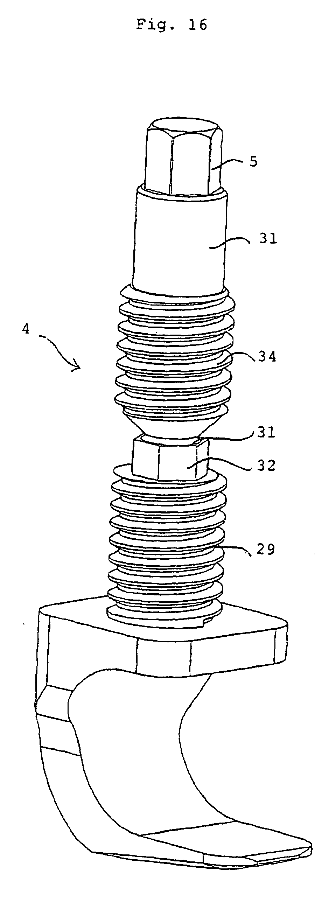

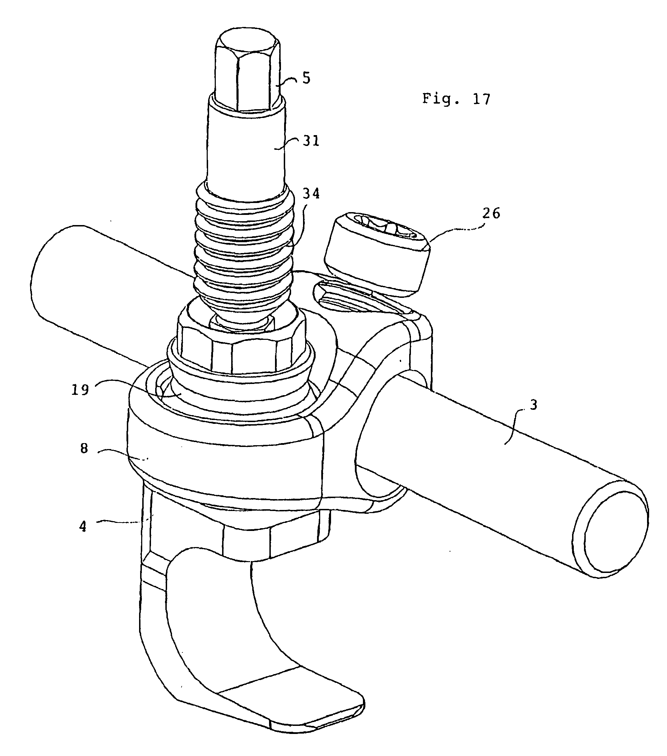

Le moyen d'ancrage osseux peut également être constitué par un crochet (4), comme illustré figures 16 et 17.

## Revendications

1. Ensemble de connexion pour l'ostéosynthèse rachidienne comportant un moyen d'ancrage osseux comprenant une zone de connexion (29) destinée à coopérer avec un moyen de connexion, **caractérisé en ce que** le moyen de connexion comporte dans sa partie inférieure une forme sphérique (20) pour permettre un positionnement libre du moyen de connexion dans un connecteur (8) ou dans un élément de liaison (3), cette forme sphérique (20) formant une butée de positionnement longitudinale avec le connecteur (8) ou avec l'élément de liaison (3, 3'), **caractérise en ce que** ledit connecteur (8) ou élément de liaison (3) présente une cavité de forme complémentaire.

2. Ensemble de connexion pour l'ostéosynthèse selon la revendication 1, **caractérisé en ce que** le moyen de connexion est serti dans le connecteur (8) de façon à solidariser les deux pièces tout en laissant une rotation du moyen de connexion dans le connecteur (8).

3. Ensemble de connexion pour l'ostéosynthèse selon la revendication 1 ou 2, **caractérisé en ce que** le connecteur présente un cône (22) d'entrée dans le connecteur (8) pour le passage de l'ancillaire pour l'entraînement en rotation du moyen de connexion.

4. Ensemble de connexion pour l'ostéosynthèse selon la revendication 3, **caractérisé en ce que** le débattement angulaire du connecteur (8) sur le moyen de connexion est de 30 degrés environ quand l'ancillaire d'entraînement en rotation du moyen de connexion est en place.

5. Ensemble de connexion pour l'ostéosynthèse selon l'une au moins des revendications précédentes, **caractérisé en ce que** le moyen de connexion présente des fentes (23) usinées dans la partie sphérique (20) du moyen de connexion de façon à créer une déformation lors du serrage définitif du système.

6. Ensemble de connexion pour l'ostéosynthèse selon la revendication 5, **caractérisé en ce que** lesdites fentes (23) sont longitudinales. (30)

7. Ensemble de connexion pour l'ostéosynthèse selon l'une au moins des revendications précédentes, **caractérisé en ce que** ledit moyen de connexion comporte dans sa partie inférieure une jupe (28).

8. Ensemble de connexion pour l'ostéosynthèse selon la revendication 7, **caractérisé en ce que** ladite jupe (28) est filetée sur son extrémité inférieure pour faciliter sa pénétration dans l'os.

9. Ensemble de connexion pour l'ostéosynthèse selon l'une au moins des revendications précédentes, **caractérisé en ce que** le moyen d'ancrage osseux comporte dans sa partie supérieure une partie lisse (30) de pré-guidage dudit moyen de connexion.

10. Ensemble de connexion pour l'ostéosynthèse selon l'une au moins des revendications précédentes, **caractérisé en ce que** le moyen de connexion est constitué par un écrou (19).

11. Ensemble de connexion pour l'ostéosynthèse selon l'une au moins des revendications précédentes, **caractérisé en ce que** le connecteur (8) est pourvu d'au moins un emplacement (24) pour recevoir un élément de liaison (3, 3').

12. Ensemble de connexion pour l'ostéosynthèse selon la revendication 11, **caractérisé en ce que** ledit connecteur (8) comporte un emplacement de blocage (25) débouchant dans la cavité formant le logement de la forme sphérique (20) et dans l'emplacement (24) recevant l'élément de liaison (3, 3').

13. Ensemble de connexion pour l'ostéosynthèse selon la revendication 12, **caractérisé en ce qu'**il comporte un cylindre de blocage (36) qui peut être introduit dans ledit emplacement de blocage (25).

14. Ensemble de connexion pour l'ostéosynthèse selon la revendication 13, **caractérisé en ce que** ledit cylindre de blocage (36) peut être remplacé par un élément de liaison (3).

15. Ensemble de connexion pour l'ostéosynthèse selon la revendication 13 ou la revendication 14, **caractérisé en ce que** ledit emplacement de blocage (25) débouche également dans une direction sensiblement perpendiculaire pour l'introduction d'un bouchon de blocage (37).

16. Ensemble de connexion pour l'ostéosynthèse selon la revendication 15, **caractérisé en ce que** ledit bouchon de blocage (37) comporte un téton (38) destiné à coopérer avec un emplacement ménagé dans ledit cylindre de réglage (36).

17. Ensemble de connexion pour l'ostéosynthèse selon l'une quelconque des revendications 11 à 16, **caractérisé en ce que** ledit emplacement (24) est en forme de oblong dans le cas d'un connecteur (8) de type fermé, ou en forme de « U » débouchante sur une des faces dans le cas d'un connecteur de type ouvert.

18. Ensemble de connexion pour l'ostéosynthèse selon la revendication 11, **caractérisé en ce que** le connecteur (8) est pourvu de deux emplacements (24) pour recevoir un élément de liaison (3') formé par une tige coudée formant un U fermé à ses extrémités et **en ce que** l'élément de liaison (3') présente une cavité de forme complémentaire à la forme sphérique (20).

19. Ensemble de connexion pour l'ostéosynthèse selon l'une au moins des revendications précédentes, **caractérisé en ce qu'**une partie filetée (40), en forme d'hexagone, est aménagée à l'extrémité supérieure du moyen de connexion, afin de permettre de visser un écrou secondaire (41).

## Claims

1. Connection assembly for spinal osteosynthesis comprising an osseous anchor means including a connection area (29) designed to cooperate with a connection means, **characterised in that** the bottom part of the connection means comprises a spherical shape (20) so that a connection means can be freely positioned in a connector (8) or in a connection element (3'), this spherical shape (20) forming a longitudinal positioning stop with the connector (8) or with the connection element (3'), **characterised in that** the said connector (8) or connection element (3') has a complementary shaped cavity.

2. Connection assembly for osteosynthesis according to claim 1, **characterised in that** the connection means is crimped in the connector (8) so as to fix the two parts while allowing the connection means to rotate in the connector (8).

3. Connection assembly for osteosynthesis according to claim 1 or 2, **characterised in that** the connector has an input cone (22) in the connector (8) to enable an ancillary to pass through to drive the connection means in rotation.

4. Connection assembly for osteosynthesis according to claim 3, **characterised in that** the angular displacement of the connector (8) on the connection means is about 30 degrees when the rotation drive ancillary of the connection means is in place.

5. Connection assembly for osteosynthesis according to at least one of the previous claims, **characterised in that** the connection means has slits (23) machined in the spherical part (20) of the connection means so as to create a deformation during final clamping of the system.

6. Connection assembly for osteosynthesis according to claim 5, **characterised in that** the said slits (23) are longitudinal.

7. Connection assembly for osteosynthesis according to at least one of the previous claims, **characterised in that** the bottom part of the said connection means comprises a skirt (28).

8. Connection assembly for osteosynthesis according to claim 7, **characterised in that** the said skirt (28) is threaded at its lower end to facilitate penetration into the bone.

9. Connection assembly for osteosynthesis according to at least one of the previous claims, **characterised in that** the top part of the osseous anchor means comprises a smooth pre-guiding part (30) for the said connection means.

10. Connection assembly for osteosynthesis according to at least one of the previous claims, **characterised in that** the connection means comprises a nut (19).

11. Connection assembly for osteosynthesis according to at least one of the previous claims, **characterised in that** the connector (8) is provided with at least one position (24) to hold a connection element (3, 3').

12. Connection assembly for osteosynthesis according to claim 11, **characterised in that** the said connector (8) comprises a blocking position (25) opening up in the cavity forming the housing of the spherical shape (20) and in the position (24) into which the connection element (3,3') will fit.

13. Connection assembly for osteosynthesis according to claim 12, **characterised in that** it comprises a blocking cylinder (36) that can be inserted in the said blocking position (25).

14. Connection assembly for osteosynthesis according to claim 13, **characterised in that** the said blocking cylinder (36) may be replaced by a connection element (3).

15. Connection assembly for osteosynthesis according to claim 13 or claim 14, **characterised in that** the said blocking position (25) also opens up in an approximately perpendicular direction for insertion of a blocking plug (37).

16. Connection assembly for osteosynthesis according to claim 15, **characterised in that** the said blocking plug (37) comprises a pin (38) that will cooperate with a position formed in the said blocking cylinder (36).

17. Connection assembly for osteosynthesis according to any one of claims 11 to 16, **characterised in that** the said position (24) is in the form of an oblong in the case of a closed type connector (8), or in the form of a "U" opening up on one of the faces in the case of an open type connector.

18. Connection assembly for osteosynthesis according to claim 11, **characterised in that** the connector (8) is provided with two positions (24) to contain a connection element (3') formed by a cranked rod forming a U closed at its ends and **in that** the shape of the connection element (3') is complementary to the spherical shape (20).

19. Connection assembly for osteosynthesis according to at least one of the previous claims, **characterised in that** a hexagonal shaped threaded part (40) is formed at the top end of the connection means, so that a secondary nut (41) can be screwed in.

## Patentansprüche

1. Verbindungsset für die spinale Osteosynthese, umfassend ein Mittel zur Verankerung im Knochen, aufweisend einen Verbindungsbereich (29), der dazu bestimmt ist, mit einem Verbindungsmittel zusammenzuwirken, **dadurch gekennzeichnet, dass** das Verbindungsmittel in seinem unteren Teil eine kugelige Form (20) aufweist, um eine freie Positionierung des Verbindungsmittels in einem Verbinder (8) zu ermöglichen oder in einem Verbindungselement (3'), wobei diese kugelige Form (20) mit dem Verbinder (8) oder mit dem Verbindungselement (3') einen Anschlag zur Längspositionierung ausbildet, **dadurch gekennzeichnet, dass** der besagte Verbinder (8) oder das Verbindungselement (3') einen komplementär geformten Hohlraum aufweist.

2. Verbindungsset für die Osteosynthese nach Anspruch 1, **dadurch gekennzeichnet, dass** das Verbindungsmittel im Verbinder (8) so gefasst ist, dass die beiden Teile bei Aufrechterhaltung einer Rotation des Verbindungsmittels im Verbinder (8) verbunden werden.

3. Verbindungsset für die Osteosynthese nach Anspruch 1 oder 2, **dadurch gekennzeichnet, dass** der Verbinder einen Eingangskonus (22) in den Verbinder (8) zum Hindurchführen der Rotationsantriebshilfe des Verbindungsmittels aufweist.

4. Verbindungsset für die Osteosynthese nach Anspruch 3, **dadurch gekennzeichnet, dass** der Winkelspielraum des Verbinders (8) auf dem Verbindungsmittel ca. 30 Grad beträgt, wenn die Rotationsantriebshilfe des Verbindungsmittels platziert ist.

5. Verbindungsset für die Osteosynthese nach mindestens einem der vorhergehenden Ansprüche, **dadurch gekennzeichnet, dass** das Verbindungsmittel Schlitze (23) hat, die in den kugeligen Teil (20) des Verbindungsmittels eingearbeitet sind, so dass bei der endgültigen Fixierung des Systems eine Deformation erfolgt.

6. Verbindungsset für die Osteosynthese nach Anspruch 5, **dadurch gekennzeichnet, dass** die besagten Schlitze (23) längs verlaufen.

7. Verbindungsset für die Osteosynthese nach mindestens einem der vorhergehenden Ansprüche, **dadurch gekennzeichnet, dass** das besagte Verbindungsmittel in seinem unteren Teil einen Mantel (28) aufweist.

8. Verbindungsset für die Osteosynthese nach Anspruch 7, **dadurch gekennzeichnet, dass** der besagte Mantel (28) an seinem unteren Ende mit einem Gewinde versehen ist, um sein Eindringen in den Knochen zu erleichtern.

9. Verbindungsset für die Osteosynthese nach mindestens einem der vorhergehenden Ansprüche, **dadurch gekennzeichnet, dass** das Mittel zur Verankerung im Knochen in seinem oberen Teil einen glatten Teil (30) zur vorbereitenden Führung des besagten Verbindungsmittels aufweist.

10. Verbindungsset für die Osteosynthese nach mindestens einem der vorhergehenden Ansprüche, **dadurch gekennzeichnet, dass** das Verbindungsmittel von einer Mutter (19) gebildet wird.

11. Verbindungsset für die Osteosynthese nach mindestens einem der vorhergehenden Ansprüche, **dadurch gekennzeichnet, dass** der Verbinder (8) mit mindestens einer Stelle (24) zur Aufnahme eines Verbindungselements (3, 3') versehen ist.

12. Verbindungsset für die Osteosynthese nach Anspruch 11, **dadurch gekennzeichnet, dass** der besagte Verbinder (8) eine Blockierstelle (25) aufweist, die in den die Aufnahme der kugeligen Form (20) bildenden Hohlraum führt und in die das Verbindungselement (3, 3') aufnehmende Stelle (24).

13. Verbindungsset für die Osteosynthese nach Anspruch 12, **dadurch gekennzeichnet, dass** es einen Blockierzylinder (36) umfasst, der in die besagte Blockierstelle (25) eingeführt werden kann.

14. Verbindungsset für die Osteosynthese nach Anspruch 13, **dadurch gekennzeichnet, dass** der besagte Blockierzylinder (36) durch ein Verbindungselement (3) ersetzt werden kann.

15. Verbindungsset für die Osteosynthese nach Anspruch 13 oder nach Anspruch 14, **dadurch gekennzeichnet, dass** die besagte Blockierstelle (25) zur Einführung eines Blockierstopfens (37) ebenfalls in eine deutlich senkrechte Richtung führt.

16. Verbindungsset für die Osteosynthese nach Anspruch 15, **dadurch gekennzeichnet, dass** der besagte Blockierstopfen (37) einen Zapfen (38) aufweist, der dazu bestimmt ist, mit einer in den besagten Blockierzylinder (36) eingearbeiteten Stelle zusammenzuwirken.

17. Verbindungsset für die Osteosynthese nach einem der Ansprüche 11 bis 16, **dadurch gekennzeichnet, dass** die besagte Stelle (24) bei einem geschlossenen Verbinder (8) länglich oder bei einem offenen Verbinder U-förmig auf eine der Seiten mündend geformt ist.

18. Verbindungsset für die Osteosynthese nach Anspruch 11, **dadurch gekennzeichnet, dass** der Verbinder (8) mit zwei Stellen (24) zur Aufnahme eines Verbindungselements (3') versehen ist, das von einer gekrümmten, an ihren Enden ein geschlossenes U bildenden Stange gebildet wird, und dadurch, dass das Verbindungselement (3') einen Hohlraum aufweist, der zu der kugeligen Form (20) komplementär geformt ist.

19. Verbindungsset für die Osteosynthese nach mindestens einem der vorhergehenden Ansprüche, **dadurch gekennzeichnet, dass** am oberen Ende des Verbindungsmittels ein sechseckiger Gewindeteil (40) ausgearbeitet ist, um eine zweite Mutter (41) anschrauben zu können.
